# EUROPEAN PATENT SPECIFICATION

(11) **EP 1 766 411 B1**
(45) Date of publication and mention of the grant of the patent: **23.02.2011**
(21) Application number: 05748087.3
(22) Date of filing: 24.05.2005
(51) Int. Cl.: G01N 33/68, C07K 14/78, C07K 16/18, C12N 15/11

(54) **METHOD FOR DETERMINING A TISSUE DEGRADATION PROCESS BY DETECTION OF COMP NEOEPITOPES**
VERFAHREN ZUR BESTIMMUNG EINES GEWEBEABBAUVERFAHRENS DURCH NACHWEIS VON COMP-NEOEPITOPEN
METHODE DE DETERMINATION D'UN PROCESSUS DE DEGRADATION TISSULAIRE PAR DETECTION DE NEOEPITOPES COMP

(30) Priority: 24.05.2004 DK 200400815
(43) Date of publication of application: 28.03.2007
(73) Proprietor: AnaMar AB, 411 36 Goteborg (SE)
(72) Inventor: HEINEGÅRD, Dick, S-226 47 Lund (SE); ÖNNERFJORD, Patrik, S-214 21 Malmö (SE); STUBENDORFF, Johann, Jakob, 23911 Gross Disnack (DE)
(74) Representative: Webber, Philip Michael
(86) International application number: PCT/EP2005/005706
(87) International publication number: WO 2005/116658

(56) References cited:
- WO-A-98/07035
- PFEIFFER F R ET AL: "Tri- and tetrapeptide analogues of kinins as potential renal vasodilators." JOURNAL OF MEDICINAL CHEMISTRY. MAR 1984, vol. 27, no. 3, March 1984 (1984-03), pages 325-341, XP002368169 ISSN: 0022-2623
- DATABASE REGISTRY Chem.Abstr.; RN=416977-96-3 16 May 2002 (2002-05-16), XP002351927 & WO 01/77384 A (EPIGENOMICS AG; OLEK, ALEXANDER; PIEPENBROCK, CHRISTIAN; BERLIN, KURT) 18 October 2001 (2001-10-18)
- VILIM V ET AL: "CHARACTERIZATION OF MONOCLONAL ANTIBODIES RECOGNIZING DIFFERENT FRAGMENTS OF CARTILAGE OLIGOMERIC MATRIX PROTEIN IN HUMAN BODY FLUIDS" ARCHIVES OF BIOCHEMISTRY AND BIOPHYSICS, NEW YORK, US, US, vol. 341, no. 1, 1 May 1997 (1997-05-01), pages 8-16, XP002050543 ISSN: 0003-9861 cited in the application
- STRACKE^A J O ET AL: "Matrix metalloproteinases 19 and 20 cleave aggrecan and cartilage oligomeric matrix protein (COMP)" FEBS LETTERS, ELSEVIER SCIENCE PUBLISHERS, AMSTERDAM, NL, vol. 478, no. 1-2, 28 July 2000 (2000-07-28), pages 52-56, XP004337405 ISSN: 0014-5793
- FOSANG A J ET AL: "Neoepitopes as biomarkers of cartilage catabolism." INFLAMMATION RESEARCH : OFFICIAL JOURNAL OF THE EUROPEAN HISTAMINE RESEARCH SOCIETY ... [ET AL.]. JUN 2003, vol. 52, no. 7, June 2003 (2003-06), pages 277-282, XP002351924 ISSN: 1023-3830
- CARRINO DAVID A ET AL: "Age-related changes in the proteoglycans of human skin. Specific cleavage of decorin to yield a major catabolic fragment in adult skin." THE JOURNAL OF BIOLOGICAL CHEMISTRY. 9 MAY 2003, vol. 278, no. 19, 9 May 2003 (2003-05-09), pages 17566-17572, XP002351925 ISSN: 0021-9258

## Description

### Field of Invention

The present Invention relates to a method for determining a tissue degradation process by detection of COMP (Cartilage Oligomeric Matrix Protein) neoepitopes, to antibodies against COMP neoepitopes and to fragments containing COMP neoepitopes for generation of such antibodies.

### Background

Pathological conditions resulting in tissue degradation such as cartilage degradation constitute a major medical, social and economical problem. Of persons older than 65 years of age, about 500 out of 1,000 have arthritis. Tissue degradation processes are characterized by the break down of tissue components. The tissue components can be degraded due to e.g. mechanical stress, toxic compounds or by enzymes. For this reason, determination of tissue degradation processes for the purpose of diagnosis, disease monitoring, treatment etc. can be performed by numerous methods. One way to determine degradation processes in connective tissue diseases, such as arthritic conditions; arteriosclerosis, degenerative joint conditions etc. is the detection of the presence of degradation products of the connective tissue components. This allows direct detection of the degradation process, compared to Indirect methods as e.g. measuring increased amounts of leukocytes, which have been widely employed in the diagnosis of inflammatory processes such as arthritic conditions.

Traditionally, the clinical diagnosis of arthritis is based on the patient's history, physical examination, and radiographs. The prognosis, treatment and clinical outcome of patients with arthritis are assessed by serial determinations. However, in order to minimise permanent tissue damage caused by pathological conditions involving cartilage degeneration, it is important to be able to diagnose such conditions at an early stage. Accordingly, during the last decade efforts have been made in order to find suitable biological markers that enable an early detection of pathological cartilage degeneration.

One such biological marker is COMP. Elevated serum level of COMP has previously been associated with joint destruction in rheumatoid arthritis (Mänsson et al., J. Clin. Invest (1995), vol. 95, pp. 1077-1077: Wolhein et al., British Journal of Rheumatology (1997), vol. 36, pp 847-849; Petersson et al., British Journal of Rheumatology (1998), vol 37, pp 46-50). Significant amounts of small fragments of COMP have also been found in synovial fluid from patients with rheumatoid arthritis and other forms of inflammatory arthritis (Neidhardt et al., British Journal of Rheumatology (1997), vol. 36, pp 1151-1160).

WO 9807035 discloses a method for the determination of tissue degradation by screening for the presence of one or more neoepitopes of COMP that appear after cleavage of COMP.

### Summary of invention

The inventors of the present invention have developed a method for the determination of tissue degradation processes by using specific fragments of COMP as a marker for tissue degradation. By using fragments of COMP as a marker for tissue degradation processes, a much higher signal to noise ratio is obtained and thereby the problem having false positive test results are minimized or almost avoided. The higher signal to noise ratio of the present method also allows earlier detection of the tissue degradation process, which is an important factor in diagnosis of degenerative conditions, as this allows early treatment of the disease and thereby a much higher chance for recovery.

Based on the identification of specific cleavage sites in COMP, the inventors have developed an improved method for the determination of a tissue degradation process. Accordingly, the present invention relates to a method for determining a tissue degradation process comprising detecting in a sample the presence of one or more neoepitopes of mammalian COMP that appear after cleavage of COMP between position 625 (N⁶²⁵) and 626 (P⁶²⁶) of the amino acid sequence of COMP (SEQ ID No: 1). The method can be used for diagnosis, disease monitoring and/or therapeutic monitoring of the tissue degradation process. The degradation process can take place in a connective tissue, such as e.g. cartilage, tendon, ligaments, bone, blood vessels, intervertebral discs and meniscus.

The detection of COMP fragment specific neoepitopes can be performed by using antibodies against the COMP neoepitopes, thus the present invention also relates to such antibodies and the use thereof. Furthermore the invention discloses isolated fragments of COMP comprising one or more neoepitopes, for use in the production of antibodies against COMP neoepitopes as defined herein.

### Detailed Description

Degradation of structures in articular cartilage is seen typically in all diseases resulting chronically in the destruction of the joint structure. As examples of such disorders may be mentioned rheumatoid arthritis, psoriatic arthritis, and osteoarthritis/osteoarthrosis. Acute inflammation of a joint e.g. in reactive arthritis, trauma etc, is often accompanied by a destruction of cartilage, although in most cases this will not develop into the chronically destructive disease. It is not known which factors are crucial for the acutely inflamed joint to either proceed to healing or develop into the chronic process. Examples of diseases involving acute joint inflammation such as reactive arthritis secondary to infection by yersinia, chlamydia, salmonella, as well as pyrophosphate arthritis, gout (Arthritis urica), septic arthritis and various arthritides of traumatic etiology. Among other factors potentially conducive to the destruction of articular cartilage may be mentioned, for instance, treatment with cortisone; this has been considered for a long time to accelerate the degenerative process in osteoarthiritis/osteoarthrosis. It should be noted, however, that the actual conditions prevailing in cases of arthritis with severe inflammation of the joint are of a rather more complex character, since in those cases injection of cortisone appears to have an overall positive effect.

Other diseases associated with tissue changes are inflammatory joint diseases such as, e.g., juvenile chronic arthritis, systemic lupus erythematosus (SLE), infectious arthritis and juvenile idiopathic arthritis, other diseases involving joint structures such as, e.g., osteoarthritis, meniscal damage, gout, diseases involving the intervertebral disc of the spine, such as, e.g., lower back pain, diseases affecting structural elements of the musculoskeletal system such as, e.g., tendon damage, ligament damage, and bone disease such as, e.g., osteoporosis, diseases affecting major arteries including atherosclerosis, vasculitis and trauma leading to injury, lung diseases including asthma and COPD.

An important object of the present invention is to improve the diagnostic possibilities of tissue degradation processes, particularly in early stages, and to provide a means of monitoring the effects of therapeutical measures taken. In particular it is envisaged that a method according to the present invention may make it possible to differentiate between tissue degradation processes like e.g. rheumatoid arthritis (RA) and osteoarthritis/osteoarthrosis (OA).

As described above, the inventors of the present invention have developed a reliable method for the determination of tissue degradation processes by measuring the presence of COMP fragments in the tissue and/or in the fluids in direct or indirect contact with the tissue. The present method allows a reliable and early detection of the tissue degradation process, which is an important factor in diagnosis of degenerative conditions, as this allows early treatment of the disease and thereby a much higher chance for recovery. The early detection of the tissue degradation process also allows monitoring of therapy efficiency, and identification of patients with an active tissue degradation process, which is suitable for therapy.

The method is based on the observation that COMP neoepitopes are formed during e.g. cartilage degradation. The delineation of specific fragments of COMP by the inventors allows identification of COMP neoepitopes, which can be used in the determination of the tissue degradation. The COMP fragments containing COMP neoepitopes can be employed in the production of antibodies against COMP neoepitopes, which may be used in the detection of the presence of COMP neoepitopes in tissue degradation processes.

### COMP fragments

COMP (Cartilage Oligomeric Matrix Protein) also referred to as Thrombospondin 5, is prominent in cartilage, intervertebral disc but is also present in tendon (Cartilage matrix proteins: An acidic oligomeric protein (COMP) detected only in cartilage. E. Hedborn, P. Antonsson. A. Hjerpe, D. Aeschlimann, M. Paulsson, E. Rosa-Pimentel, Y. Sommarin, M. Wendel, A. Oldberg and D. Heinegård. J. Biol. Chem. (1992) 267, 6132-6136., The distribution of cartilage oligomeric matrix protein (COMP) in tendon and its variation with tendon site, age and load. R. Smith, L. Zunino, P. Webbon and D. Heinegård. Matrix Biology (1997) 16, 255-271).

The protein has a role in formation of the collagen network, an important structural entity in cartilage. Thus, it has been shown to bind to collagen with high affinity (Cartilage oligomeric matrix protein shows high affinity. Zn-dependent interaction with triple helical collagen. K. Rosenberg, H. Olsson, M. Mörgelin and D. Heinegård. J. Biol. Chem. (1998) 273, 20397-20403.). In mutations of the protein severe growth disturbances of pseudoachondroplasia and multiple ephiphyseal dysplasia are observed (Mutations in Cartilage Oligomeric Matrix Protein causing Pseudoachondroplasia and Multiple Epiphyseal Dysplasia affect binding of Calcium and Collagen I, II and IX. J. Thur, K. Rosenberg, P. Nitsche, T. Pihlajamaa, L. Ala-Kokko, D. Heinegård, M. Paulsson and P. Maurer. J. Biol. Chem. (2001) 276, 6083-6092.).

In a variety of joint disease conditions the protein is fragmented and released from the cartilage to the synovial fluid, where the fragments can be detected (Cartilage oligomeric matrix protein. A novel marker of cartilage turnover detectable in synovial fluid and blood. T. Saxne and D. Heinegård. Brit. J. Rheumatol (1992) 31, 583-591; Cartilage and bone metabolism in rheumatoid arthritis: Differences between rapid and slow progression of disease identified by serum markers of cartilage metabolism. B. Månsson, M. lonescu, A.R. Poole, D. Heinegård and T. Saxne. J. Clin. Invest. (1995) 95, 1071-1077; Serum levels of cartilage oligomeric matrix protein (COMP) correlate with radiographic progression of knee osteoarthritis. Vilim V, Olejarova M, Machacek S, Gatterova J, Kraus VB, Pavelka K. Osteoarthritis Cartilage. 2002 Sep;10(9):707-13.). The released molecular constituents largely represent fragments, but little is known of their character albeit some mapping of the regions recognized by some of the antibodies used have been made (Monoclonal antibodies to human cartilage oligomeric matrix protein: epitope mapping and characterization of sandwich ELISA. Vilim V, Voburka Z, Vytasek R, Senolt L, Tchetverikov I, Kraus VB, Pavelka K. Clin Chim Acta. 2003 Feb;328(1-2):59-69). To date little is however known of the cleavage that generate the fragments observed.

One problem in assays of COMP in body fluids for diagnosis and monitoring of joint disease and tissue breakdown is that there is a background turnover in particularly all cartilaginous tissues that is part of the normal adaptation of these tissues to load and environment. As a result, the increased breakdown occurring in a particular joint cartilage in disease can only be seen against a background of normal turnover. Thus, the increase is only on top of an existing level. It is desirable to identify facts about the fragments that distinguishes the pathological ones from those normally present. One approach for this is to identify the specific terminals that are present in the various fragments and then develop antibodies that only recognize a specific such terminus and thereby only a given fragment.

The present inventors therefore undertook to study COMP fragments in a tissue, which is undergoing degeneration with apparent breakdown and alteration of the tissue matrix. This tissue, the intervertebral disc from elderly people was further studied.

In general COMP was extracted and purified. Fragments were separated and the terminus of two such fragments determined, thereby each defining two new epitopes (neoepitopes), that is the peptide sequence on the N-terminal side of the cleavage (defining a new C-terminus) and the peptide sequence on the C-terminal side of the cleavage (defining a new N-terminus). Antibodies have been raised to one of these peptide sequences as demonstrated to react with the fragment, while not with the intact COMP.

### Definitions

The term "tissue degradation process" is defined as any stage from the beginning to the end of a break down process in tissue.

The term "neoepitope" is defined as an epitope, which is not normally exposed on normal tissue, cells or molecules. Phenotypic changes in a cell may include loss of normal cell surface antigenic epitope components and the gain of new neoepitopes. Other ways of gaining neoepitopes are by structural changes of molecules, such as proteins. In the context of this application, a neoepitope is an epitope that is not normally exposed on COMP, but appears after changes of the protein. Examples of changes may be alterations in folding of the protein resulting in structural changes of the secondary and/or tertiary structure of COMP. A change of COMP may also result from e.g. cleavage of the protein. The neoepitope may for example be formed by a primary, secondary and/or tertiary structure of a COMP fragment appearing after cleavage.

The term "fragment" is defined as a degradation product of a protein generated by the break at one or more points of the protein back bone. In the current context, fragment is defined as a fragment of COMP generated by the degradation of COMP such as e.g. by cleavage of the protein. The fragment can be generated either by degradation of COMP *in vivo* or *in vitro* optionally followed by isolation of the fragment or the fragment can be generated *in vitro* by expressing parts of the COMP molecule using a synthetic, semisyntheticand/or recombinant method.

The term "peptide" is defined as a sequence of amino acids. The amino acids present in the sequence can be any natural or unnatural, substituted or unsubstituted amino acids. The peptide comprises from two to 100 amino acids in the sequence.

In the present context the term "COMP fragment" is intended to indicate a part of the full-length COMP sequence, e.g. a C-terminally and/or N-terminally truncated version thereof. A specific example of a COMP fragment contains a sequence of at least 3 amino acid residues that has a sequence identity of 100% to that of a mammalian COMP such as, e.g., human COMP as claimed herein.

The term "antibody" is defined as a compound having a structure that binds to one or more antigens and/or epitopes. In the present context, the antibody/compound is directed to neoepitopes of COMP. The compound can be one or more fragments of an antibody, such as a Fab (Fragment Antigen Binding) fragment or a F(ab)'₂ fragment (a fragment containing two Fab), or the compound can be an antibody in general.

The term "spacer" is defined as a compound with the function to keep two other compounds, molecules or atoms (e.g. a COMP fragment and an immunogenic protein) linked to each end of the spacer, apart with a certain distance. Examples of spacers are one or more amino acid residues, such as e.g. one or more glycine residues, one or several monosaccharide residues, synthetic polymers including e.g crosslinking agents with a variable long aliphatic chain with hydrophilic groups examplified by the SPDP (N-(Succinimidyl-pyridyldithio-propionate-hexanoate) reagents from Pierce Chemicals.

In the current application, the amino acids in COMP are numbered according to the sequence listing.

### Cleavage sites of COMP

The inventors of the present invention have identified hitherto unknown specific cleavage sites of COMP. The cleavages sites are located in the N-terminal of COMP. more specifically between position 530 and 660 of the amino acid sequence of COMP (SEQ ID NO.1), e.g. the cleavage of COMP is at one or more sites between position 540 and 650, such as e.g. between position 540 and 640 of the amino acid sequence of COMP.

More specifically and as shown in the examples herein, the present inventors have found two cleavage sites of human COMP. One betweeen position 540 and 560 of the amino sequence of COMP and the other between position 610 and 630 of the amino sequence of COMP.

The specific cleavage site between position 540 and 560 is between position 550 (N⁵⁵⁰) and 551 (W⁵⁵¹), whereas the other specific cleavage site is between position 625 (N⁶²⁵) and 626 (P⁶²⁶)_{.}

The above-mentioned cleavage sites relate to human COMP.

### Fragments of COMP for use in the production of antibodies

As mentioned earlier, the identification of COMP neoepitopes allows the production of antibodies against the neo-epitopes for determination of cartilage degradation. An isolated form of one or more fragments of COMP comprising neoepitopes can be used to generate such antibodies.

The desired specificity in a method according to the present invention usually requires 12 such as, e.g., 10 or less amino acid residues with the new terminus free. Thus, it is contemplated that peptides having 3 amino acid residues such as, e.g., 4, 5, 6, 7, 8, 9, 10, 11 or 12 or more amino acid residues having an identity of 100% to the C-terminal part (i.e. the last 1-15 amino acid residues) of the novel peptide fragments on the N-terminal side of the cleavage (defining a new C-terminus) or to the N-terminal part (i.e. the first 1-15 amino acid residues) of the novel peptide fragments on the C-teminal side of the cleavage (defining a new N-terminus), respectively, are useful in this context.

The fragments can occur *in vivo* as a result of COMP degradation, or the fragments can be prepared *in vitro* by enzymatic cleavage or by a synthetic, semisynthetic and/or recombinant method.

The use of COMP fragments occurring *in vivo* for the generation of antibodies against COMP neoepitopes, involves the isolation of the fragments from a body fluid such as blood or synovial fluid, from connective tissue, such a cartilage, bone, ligaments, tendon, blood vessels, intervertebral discs and meniscus. The fragment can also be isolated from cells in a connective tissue, such as e.g. fibroblasts, chondrocytes, smooth muscle cells or tumor cells.

The preparation of the fragment *in vitro* can be performed by a method for expressing the fragment, comprising the steps of
i) constructing a recombinant DNA expression vector comprising the polynucleotide sequence encoding the fragment to be expressed,
ii) transforming a compatible procaryotic or eucaryotic host cell with the vector such that the fragment can be expressed by the host cell, and culturing the transformed host cell in a suitable growth medium to produce the fragment, and
iii) optionally, isolating and/or purifying the fragment.

The fragments according to the present invention are fragments of human COMP (SEQ ID NO 1).

In the following, specific fragments are mentioned.

### Fragments of human COMP containing neoepitopes appearing after cleavage between N⁶²⁵ and P⁶²⁶ of COMP

In one embodiment, the invention relates to a fragment of human COMP, wherein the fragment has the amino acid sequence WKQMEQTYWQAN (COMP₈₁₄₋₆₂₅) (SEQ ID NO 22).

In other embodiments the invention relates to one or more fragments of human COMP, wherein the fragment has one of the following sequences:
KQMEQTYWQAN (SEQ ID NO 23)
QMEQTYWQAN (SEQ ID NO 24)
MEQTYWQAN (SEQ ID NO 25)
EQTYWQAN (SEQ ID NO 26)
QTYWQAN (SEQ ID NO 27)
TYWQAN (SEQ ID NO 28)
YWQAN (SEQ ID NO 29)
WQAN (SEQ ID NO 30)
QAN (SEQ ID NO 31)

The cleavage results in appearance of another fragment having P as a new N-terminus. Accordingly, the invention also relates to one or more fragments of human COMP, wherein the fragment has one of the following sequences:
PFRAVAEPGIQL (COMP₆₂₆₋₆₃₇) (SEQ ID NO 32)
PFRAVAEPGIQ (SEQ ID NO 33)
PFRAVAEPGI (SEQ ID NO 34)
PFRAVAEPG (SEQ ID NO 35)
PFRAVAEP (SEQ ID NO 36)
PFRAVAE (SEQ ID NO 37)
PFRAVA (SEQ ID NO 38)
PFRAV (SEQ ID NO 39)
PFRA (SEQ ID NO 40)

### Peptides and/or fragment conjugates

Peptides are usually not antigenic on their own. Therefore peptides are often coupled to a foreign protein thereby acting as a hapten. Usually, such proteins are chosen such that they do not occur in any species to be investigated to avoid reaction of the antibodies to this protein. In this way, reactivity will be restricted to the neoepitope on the short peptide.

The present invention also relates to a fragment conjugate for use in the production of antibodies against one or more COMP neoepitopes as defined herein. Preferable fragments according to the invention are coupled via the terminal that does not represent the cleavage site to a protein carrier. In a specific embodiment, the protein carrier is selected from the group consisting of keyhole limpet hemocyanin (KLH), ovalbumin and may also employ other proteins not present in the immunized animal with or without covalent coupling.

### Antibodies against COMP neoepitopes.

The observations that neoepitopes of COMP are formed upon degradation of the protein including the identification of the cleavage sites of COMP are useful in the generation of antibodies against the COMP neo-epitopes. Thus, the present invention concerns one or more antibodies raised against one or more COMP neoepitopes of the invention. Degradation of COMP results in COMP fragments that may contain neoepitopes formed by e.g. a part of the primary, secondary and/or tertiary structure of the amino acid sequence of the fragments. Accordingly, the invention also concerns COMP neoepitopes appearing in one or more fragments of COMP produced by the cleavage of COMP, between positions 625 (N⁶²⁵) and 626 (P⁶²⁶) of the amino acid sequence of COMP (SEQ ID NO:1), as defined in claims 2 - 4.

Examples of neoepitopes formed by the primary structure of the fragments are the new N- and C-terminals of COMP appearing after cleavage. The neoepitope may be formed by 3-12 amino acids of the new N- and C-terminals. Examples of the amino acid sequences of such neoepitopes are disclosed in the sections *"Fragments for use in the production of antibodies against COMP neoepitopes"* and *"Peptides for use in the production of antibodies against COMP neoepitopes".*

The neoepitopes can also be formed by the secondary structure of the COMP fragments generated by cleavage, and therefore comprise secondary structural elements, such as e.g. α-helices, β-strands, β-turns, and/or cysteine loops/disulfide bridges etc. of the fragments. Furthermore, the neoepitopes may be formed by tertiary structural elements of the COMP fragments.

The antibodies against COMP neoepitopes may be obtained by immunizing an animal with one or more fragments of COMP, and/or one or more conjugates according to the invention. The immunized animal may be selected from the group comprising humans, mice, rats, rabbits, sheep, non-human primates, goat, horse and poultry.

The antibodies according to the Invention may also be obtained by *In vitro* immunization methods using one or more fragments of COMP, and/or one or more conjugates according to the invention.

The antibody according to the invention may be a polyclonal antibody or a monoclonal antibody.

Production of an antibody according to the invention may involve the phage-display approach. When generating the antibody by the phage-display approach, the antibody may be one or more fragments of an antibody, such as a Fab (Fragment Antigen Binding) fragment or a F(ab)'₂ fragment (a fragment containing two Fab), or an intact antibody that binds to one or more neoepitopes of COMP.

The antibodies according to the invention may be used for the detection of COMP neoepitopes, and accordingly the present invention also relates to a method for determining a tissue degradation process according to the invention, wherein the presence of one or more neoepitopes is detected by using one or more antibodies according to the invention.

The antibodies against COMP neoepitopes according to the invention may be used in medicine. As mentioned above, the antibodies may be used for determining a tissue degradation process, wherein COMP neoepitopes are present. Such determination method may be employed in diagnosis, disease monitoring and/or therapeutic monitoring of a tissue degradation process.

Examples of tissue degradation processes, which may be determined by a determination method according to the invention, are tissue degradation processes involved in arthritis. The arthritis condition may be selected from the group consisting of chronic arthritis, such as e.g. rheumatoid arthritis, osteoarthritis/osteoarthrosis, psoriatic arthritis, juvenile chronic arthritis, systemic lupus erythematosus (SLE), infectious arthritis and juvenile idiopathic arthritis, diseases involving intervertebral disc of the spine, such as e.g. lower back pain, meniscal damage, tendon and ligament diseases, diseases affecting major arteries including atherosclerosis, vasculitis, trauma leading to injury and gout.

### Use of antibodies raised against one or more neoepitopes of COMP

The antibodies to these neoepitopes will be used to develop specific assays that will only detect fragments that contain these specific cleavages.

Accordingly, the use of these antibodies may be
i) in combination with other COMP-reacting antibodies in sandwich type immunoassays where one antibody, e.g. specific for the neoepitope is used to catch the fragment and another antibody. The other antibody could be an antibody reacting with the other terminus of the fragment or an antibodiy reacting with any site of present on the fragment,
ii) the use of the antibodies in inhibition immunoassays where the binding of the antibody to the specific epitope, e.g. presented by a synthetic peptide or by a fragment is inhibited by the body fluid sample to be assayed.

The assays provide a high specificity for the fragments present in the body fluids and are specific for RA and OA.

Accordingly, the antibodies of the present invention may be used in diagnosis, differential diagnosis (e.g. differentiating between two distinct diseases or between normal and diseased cartilage or other tissues), disease monitoring and/or therapeutic monitoring of a tissue degradation process.

Furthermore, in order to verify the specificity of the antibodies a removal of the new terminal amino acid of the peptide should delete the antibody reactivity. The antibody will also have to be tested against the intact protein to show lack of reactivity. Further specific fragment reactivity can be tested in Western blotting. Reactivity should be inhibited by the correct synthetic peptide but not by unrelated peptides.

One application is to raise antibodies by in vitro technology and another is to use phage display procedures to search for specific reacting peptides/proteins, not necessarily antibodies.

### Kits

The commercial form of an analytical method is often a kit. A kit includes more than one component for the performance of the assay. It may include a substance and detailed instruction for use in general, the kit includes all or most of the components necessary for the assay.

There are numerous types of methods that can be used for the analysis of a specific substance. To find the location of a substance in tissue, e.g. fluorescent or gold labeled reactive components like antibodies can be used. Different methods for detecting and quantifying a substance in various fluids include nephelometric and turbidometric methods but most particularly ELISA and RIA. All these methods have detailed names after the special setting in performance and applications. The ELISA could e.g. be either presented as a sandwich method including a catcher situation and a later stage of detection of bound substance or an inhibition method where the substance to be analyzed may in a mixture react with reactive components like antibodies and the nonreactive antibodies in this mixture are later detected after reacting with e.g. immobilized pure substance.

A kit for determining a tissue degradation process by a method according to the invention may comprise the following components:
i) A first component comprising one or more antibodies according to the invention. The antibodies can be ligands, antibody fragments, such as Fab or F(ab)'2 identified by the phage-display methods or intact antibodies binding to COMP neoepitopes.
ii) A second component comprising means for detecting whether the one and more antibodies have reacted with one or more neoepitopes as defined above.

A way to detect in a sample the presence of one or more COMP neoepitopes on fragments resulting from COMP degradation, is e.g. by a competition assay. In an example of such a competition assay, one or more antibodies according to the present invention, which bind to one or more COMP neoepitopes, are coupled to a solid phase, such as e.g. a well in a plate. The one or more antibodies are occupied with labelled fragments containing COMP neoepitopes by binding to the neoepitopes. The presence of COMP neoepitopes in a sample can then be measured by adding the sample to the well in the plate. If one or more COMP neoepitopes are present in the sample, they will compete with the labelled fragments containing COMP neoepitopes for the binding to the antibodies, and a decrease in the level of the labelling (e.g. radioactivity, fluorescence etc) can be detected.

Thus, a kit for determining a tissue degradation process by a method according to the invention may comprise the following components:
i) A first component comprising one or more antibodies according to the invention, the one or more antibodies optionally being coupled to a solid phase.
ii) A second component comprising one or more labelled fragments of COMP comprising one or more neoepitopes as defined above.

Another way to detect in a sample the presence of one or more COMP neoepitopes on fragments resulting from degradation of COMP, is e.g. by a sandwich assay comprising two different components which bind to one or more fragments containing one or more COMP neoepitopes: A first component binding to an arbitrary part of the one or more fragments, and a second component comprising one or more antibodies of the invention, which bind to the one or more COMP neoepitopes of the one or more fragments. In an example of such a sandwich assay, the first component is coupled to a solid phase, such as e.g. a well in a plate. The presence of COMP neoepitopes in a sample can then be detected by firstly adding the sample to the well. If one or more fragments containing COMP neoepitopes are present in the sample, the fragments will be bound to the solid phase via the first component, and the presence of COMP neoepitopes on the fragments can then be measuring by secondly adding the second component comprising the antibodies of the invention, which bind to the one or more COMP neoepitopes. The antibodies of the invention are optionally labelled with e.g. a radioactive or fluorescent label in order to measure the level of bound antibody to the COMP neoepitopes present in the sample. If the antibodies are not labelled, the level of bound antibodies to the COMP neoepitopes present in the sample can be measured by adding a third labelled component, which binds to antibodies.

In the sandwich assay, the component coupled to the solid phase can either be the first or the second component. In the example given above, the first component is coupled to the solid phase. In the following situation, the second component is coupled to the solid phase. In another example of the sandwich assay, the second component comprising the antibodies of the present invention, is coupled to a solid phase, such as e.g. a well in a plate. The presence of COMP neoepitopes in a sample can then be detected by firstly adding the sample to the well in the plate. If one or more fragments containing COMP neoepitopes are present in the sample, the fragments will be bound to the solid phase via the antibodies, which bind to the COMP neoepitopes on the fragments. The presence of fragments containing COMP neoepitopes can then be measured by secondly adding the first component comprising one or more substances, which bind to an arbitrary part of the one or more fragments. The one or more substances are optionally labelled with e.g. a radioactive or fluorescent label in order to measure the level of bound substance to the fragments present in the sample. If the substances are not labelled, the level of bound substance to the fragment containing COMP neoepitopes present in the sample can be measured by adding a third labelled component, which binds to the one or more substances.

Alternatively a kit for determining a tissue degradation process by a method according to the invention may comprise the following components:
i) A first component comprising one or more substances which bind to COMP or a fragment thereof, the substances optionally being coupled to a solid phase and optionally being labelled.
ii) A second component comprising one or more antibodies according to the invention, and the one or more antibodies optionally coupled to a solid phase and optionally being labelled.
iii) Optionally, a third component comprising means for detecting whether the one and more substances or the one or more antibodies have reacted with one or more neoepitopes as defined above.

It is also possible to detect in a sample the presence of one or more antibodies which bind to COMP neoepitopes as defined above, e.g. by an assay comprising two different components: A first component comprising one or more fragments of COMP comprising one or more neoepitopes as defined above, and a second component comprising means for detecting whether one and more antibodies in the sample have reacted with the neoepitopes.

In an example of the assay, the one or more fragments of COMP comprising one or more neoepitopes as defined above, are coupled to a solid phase, such as e.g. a well in a plate. The presence of antibodies in a sample can then be detected by firstly adding the sample to the well. If one or more antibodies against COMP neoepitopes are present in the sample, the antibodies will be bound to the solid phase via the one or more fragments, and the presence of antibodies can then be measuring by secondly adding the second component comprising substances, which bind to the one or more antibodies. The substances can be labelled with e.g. a radioactive or fluorescent label in order to measure the level of bound antibody to the COMP neoepitopes on the fragments in the well.

A kit for determining a tissue degradation process determining in a sample any presence of one or more antibodies against one or more neoepitopes as defined herein may also comprise the following components:
i) A first component comprising one or more fragments of COMP comprising one or more neoepitopes as defined in the claims, the one or more fragments optionally being coupled to a solid phase.
ii) A second component comprising means for detecting whether one and more antibodies have reacted with the neoepitopes.

In one embodiment, the tissue degradation process takes places in a connective tissue. The connective tissue may be selected from the group consisting of cartilage, tendon, ligaments, bone, blood vessels, intervertebral discs and meniscus.

In a second embodiment, the tissue degradation process is involved in arthritis. The arthritis may be selected from the group consisting of chronic arthritis, such as e.g. rheumatoid arthritis, osteoarthritis/osteoarthrosis, psoriatic arthritis, juvenile chronic arthritis, systemic lupus erythematosus (SLE), infectious arthritis and juvenile idiopathic arthritis, diseases involving intervertebral disc of the spine, such as e.g. lower back pain, meniscal damage, tendon and ligament damage, diseases affecting major arteries including atheroclerosis, vasculitis, trauma leading to injury and gout.

### Legends to figures

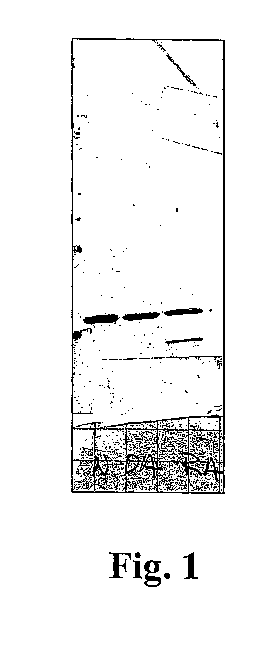

Figure 1 shows immunoblot using PFRAVAE-neoepitope antibody of normal, RA and OA sample. The characterised fragment (18 kD) is the upper band found in all samples. However, the lower band in RA sample is specific for this kind of sample and may be used as a marker for RA. The epitope of the other terminal in this case must be located before the absolute C-terminal. The C-terminal antibody did not recognize this fragment, but the 18 kD was found here.

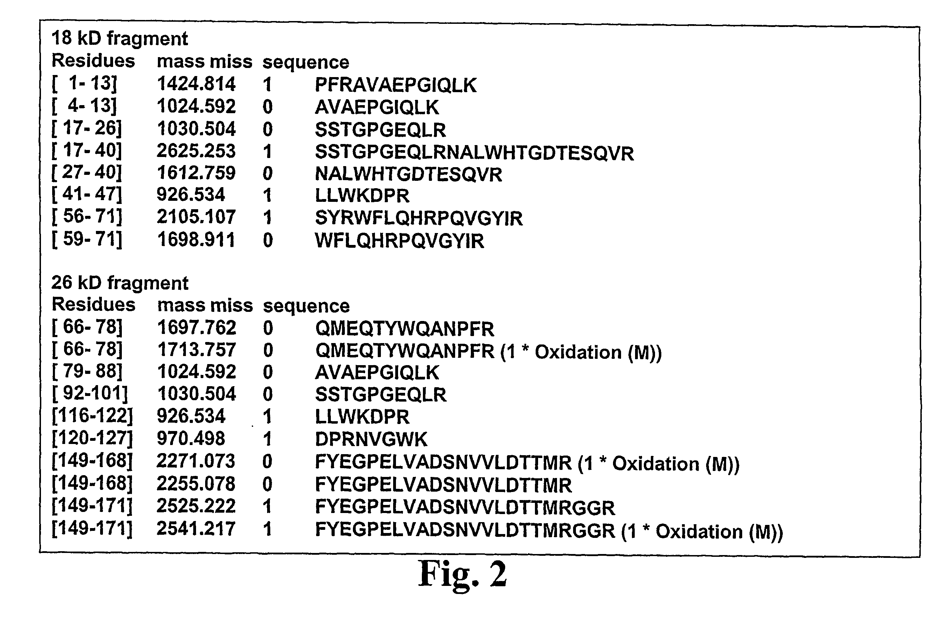

Figure 2 shows the peptides identified in trypsin digest of 18 and 26 kD COMP fragments (residues are numbered from the beginning of the fragment).

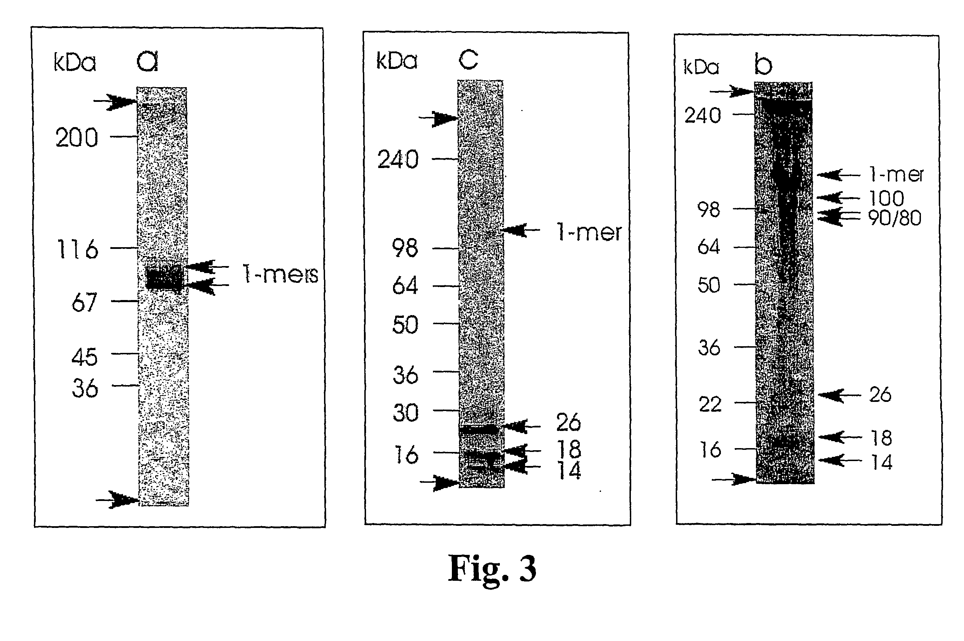

Figure 3 shows different blots to demonstrate the presence of those fragments of COMP in tissue extracts that were used for identification of neoepitopes by mass spectrometry: a) C-term anti-COMP blot (non-reduced gel); b) C-term anti-COMP blot (reduced gel); c) G-250 Brilliant Blue gel (red.,alkylated, +PNGase).

### Examples

### Example 1

### Identification of neoepitopes in human COMP

Guanidine-HCl extraction of human intervertebral disc (Anulus fibrosus and Nucleous pulposus): 20 g of tissue was cut at into small pieces of approximately 1g. Each piece of Anulus fibrosus and Nucleous pulposus was homogenised at 4°C with a polytron homogeniser for 3 x 30 s in 20 mL extraction buffer of PBS at pH 7.4, Proteins were extracted for 24 h at 4°C. Additional extraction steps with PBS pH 7.4 containing 100 mM EDTA and finally with 4 M guanidinium chloride (GuHCl) in 50 mM sodium acetate (NaOAc) pH 5.8 were made. A cocktail with standard protease inhibitors were added in all extractions. After each extraction step the samples were centrifuged for 30 min at 14000 g and the supernatant was collected, i.e. 6 supernatants were obtained. The supernatant from the first PBS extraction of Anulus fibrosus contained both intact COMP and COMP fragments and this sample was use for further characterisation.

Isolation of intact and fragmented COMP: The supernatant from the PBS extractions were loaded on a HiTrap heparin column (Pharmacia, 1 mL) and the flowthrough containing COMP and its fragments was collected. The flowthrough was further purified on a anion exchange HiTrap Q (Pharmacia, 5 mL) column with a gradient of 0.07 to 0.4 M NaCl (in 50 mM Tris HCl pH 7.4) at a flow rate of approximately 0.5 mL/min. COMP and its fragments were eluted in fraction 36-42 (≈0.29 M NaCl), which were pooled and diluted 1:3 with 50 mM Tris HCl, pH 7.4. The pooled sample was similarly purified on a HiTrap Q anion exchange column using a HPLC system (Pharmacia, SMART) and a salt gradient of 0.07 to 0.4 M NaCl in 60 min. Intact COMP eluted in fractions 63-70 while COMP fragments were eluted in fractions 55-62. Individual fractions 57-59, 65 and 70 were then subjected to a gel filtration column SuperDex 200 using a running buffer of 4 M GuHCl in 50 mM NaOAc pH 5.8. Fractions containing COMP (33-36) and COMP fragments (43-47) were pooled separately and run on the SMART system using reversed phase chromatography with a SC-C2/C18 Pharmacia column (2.1 x 100 mm). A gradient of 0-100 % acetonitrile in 0.1 % TFA was run in 60 min. SDS-PAGE (Lemmli, 1970) and immunoblotting (antibodies against human c-terminal COMP) was used as a tool in order track COMP during the purification steps.

### Sample preparation for mass spectrometry:

Samples were treated with N-glycosidase F (Boehringer-Mannheim) to remove N-linked glycosylations. N-glycosidase F digestion was performed in 25 mkM ammonium bicarbonate pH 8.0 at 37°C for 12-16 hours. Samples were applied to 4-16 % SDS-PAGE gradient gels. Gels were fixed in 40% v/v methanol, 7% v/v acetic acid for one hour and stained with Coomassie Brilliant Blue G-250 (Serva) or silver nitrate (Shevchenko et al. Anal Chem. 1996 Mar 1;68(5):850-8). Following de-staining with distilled water, strongly staining bands were excised and washed with 400 µl of water for one hour. The gel pieces were then de-stained with four 300 µl washes with 40% v/v acetonitrile in 25 mM NH₄HCO₃ for 15 minutes each until they were visibly colourless. They were then dried in a Speed vac concentrator. Samples were re-hydrated in 40 µl of 10 mM dithiothreitol in 25 mM NH₄HCO₃. Reduction of proteins was carried out at 56°C for 30 min. Superfluous solution was removed and proteins were alkylated with 40 µl of 50 mM iodoacetamide in 25 mM NH₄HCO₃ and incubated in complete darkness at room temperature. The gel pieces were then washed three times with 40% v/v acetonitrile in 25 mM NH₄HCO₃ and evaporated to complete dryness.

Identification of COMP fragment by mass spectrometry: Proteins were degraded into characteristic fragments with either trypsin (Sequencing grade, Promega), endoproteinase Glu-C or endoproteinase Lys-C (Boehringer-Mannheim). Briefly, gel pieces were re-hydrated in 12 µl of respective enzyme solutions 20-25 µg/ml in 25 mM NH₄HCO₃. Following re-hydration, 23 µl of the same buffer was added and samples were incubated at 37°C for 16-24 hours. For Glu-C digestion PBS was used instead of NH₄HCO₃. The gel pieces were then extracted with 2% v/v trifluoroacetic acid in water. Peptide containing solutions were then purified on C18 Ziptips (Millipore) with subsequent elutions at 20, 50 and 70 percent trifluoroacetic acid made up in saturated α-cyano-4-hydroxycinnamic acid (Sigma). One-microlitre aliquots of elution solution/matrix mixtures were spotted directly onto a MALDI target plate and allowed to air dry. The MALDI target plate was loaded into a Bruker Reflex™ III mass spectrometer. The polarity of the instrument was set for positive ions using delayed extraction and with the detector in reflector mode. The acceleration voltage was set at 26 kV and 120 shots per sample spot were summed in each spectrum. Protease autolysis products were used as reference masses. Spectra were analysed for matches using the ProFound search engine (Rockefeller University) (Zhang et al. 2000).

### Result:

COMP fragment identification and characterisation: In order to determine the identity of the faster migrating bands reactive with the anti-COMP c-terminal antibody, migrating at 18 and 26 kD (see figure 3), in gel digestion followed by mass spectrometry was used. The peptide sequence coverage of the bands identified them as C-terminal fragments of human COMP. In order to map and identify the new N-terminal formed in these fragments several different proteases were used. Peptides identified in 18 kD and 26 kD bands are shown in Figure 3.

When the trypsin digestes fragment is compared to the sequence coverage of intact human COMP that was run in parallel as a control, the peptide "QMETYWQANPFRAVAEPGIQLK" residues 617-638 was only found in the intact COMP sample. The result indicated that the new epitope is located within this sequence. When searching the unmatched peptides according to this sequence a mass match appears showing that the new N-terminal is "PFRAVAEPGIQLK" aa 626-638. The unmatched experimental mass was 1425.5 Da and tandem mass spectrometry using post-source decay (PSD) fragmentation was performed for this peptide supporting this sequence. In addition, the corresponding peptide was found also in the Glu-C (PFRAVAE) and Lys-C (PFRAVAEPGIQLK) digested sample. The 18 kD fragment was also submitted for conventional N-terminal sequencing (Bo Ek, SLU) and the results also supported the suggested sequence 9 out of 12 residues matched the first or second choice of suggestion. Two residues gave no results.

The 26 kD band similarly showed a new c-terminus at aa 550-551 (N/W) since the tryptic digest of the fragment resulted in identification of the non-tryptic peptide 551-558 (N)/ WVVLNQGR was identified. In addition, the corresponding peptide 551-559 WVVLNQGRE was found in the Glu-C digest of the fragment.

### Example 2

### Neo-epitope antibody production and characterisation

### Method:

Neo-epitope antibody production: The new N-terminal amino acid sequence PFRAVAE was used to synthesise a peptide to which a C-terminal cysteine was added for coupling purposes. This peptide was coupled to BSA, KLH and thiopropyl Sepharose, respectively (Schafer-N, Copenhagen, Denmark).

Five hundred micrograms of KLH conjugate was dissolved in 500 µl of PBS and then emulsified with an equal volume of complete Freund's adjuvant. New Zealand white rabbits were injected with a 500 µg dose followed by a booster injection four weeks later. Booster injections were prepared in the same manner though with incomplete adjuvant. Test bleeds were taken two to three weeks after the first boost.

Affinity purification of NeoCOMP antiserum: A six hundred microlitre (packed bed volume) column of NeoCOMP conjugated to thiopropyl sepharose was prepared in a BioRad column fitted with a 22 gauge needle to slow down the flow rate. The column was washed with 4 ml of PBS. One millilitre of crude serum was allowed to drip through. The flow through was recovered and passed through three times more. Four millilitres of PBS containing 1M NaCl was used to wash away loosely bound material. The column was then washed with 4 ml of PBS. Specific antibodies were eluted with 100 mM Glycine-HCl, pH 2.8 and 250 µl fractions were neutralised by collection into Eppendorf tubes containing 12.5 µl of 100 mM Tris-HCl, pH 9.5.

### Testing of neo-epitope antibody specificity by ELISA:

Testing of neo-epitope antibody specificity by Western blotting: Specificity for the cleaved form of COMP and confirmation of the neo-epitope was performed by Western blotting.

### Results:

A neo-epitope antibody raised to the synthetic peptide was found to be reactive only with the cleaved form of COMP. However, immunoblots of OA and RA samples resulted in various fragments detected in OA vs RA. No intact COMP was detected with the antibody.
OA - 60 kD
RA-14kD
One larger fragment was found in OA, RA and normal

## Claims

1. A method for determining a tissue degradation process comprising detecting in a sample the presence of one or more neoepitopes of mammalian COMP that appear after cleavage of COMP between position 625 (N⁶²⁵) and 626 (P⁶²⁶) of the amino acid sequence of COMP (SEQ ID NO: 1).

2. An isolated C-terminally truncated fragment of human COMP wherein the C-terminus of the fragment is amino acid 625 of SEQ ID NO: 1, and wherein the fragment comprises an amino acid sequence given in any one of SEQ ID NOs: 22-31.

3. An isolated N-terminally truncated fragment of human COMP wherein the N-terminus of the fragment is amino acid 626 of SEQ ID NO: 1, and wherein the fragment comprises an amino acid sequence given in any one of SEQ ID NOs: 32-40.

4. An isolated fragment of human COMP consisting of the amino acid sequence given in any one of SEQ ID NOs: 22-40.

5. Use of a fragment of COMP as defined in any one of claims 2 to 4 for the production of antibodies against one or more COMP neoepitopes that appear after cleavage of COMP between position 625 (N⁶²⁵) and 626 (P⁶²⁶) of the amino acid sequence of COMP (SEQ ID NO: 1).

6. A conjugate for use in the production of antibodies against one or more COMP neoepitopes that appear after cleavage of COMP between position 625 (N⁶²⁵) and 626 (P⁶²⁶) of the amino acid sequence of COMP (SEQ ID NO: 1), consisting of (i) one or more fragments as defined in claim 4 coupled to or admixed with a protein carrier, or (ii) one or more fragments as defined in claim 2 or claim 3, coupled via the terminus that does not represent the cleavage site to a protein carrier.

7. An antibody against an isolated fragment of COMP as claimed in any one of claims 2 to 4 or a conjugate as claimed in claim 6 wherein the antibody binds to one or more COMP neoepitopes that appear after cleavage of COMP between position 625 (N⁶²⁵) and 626 (P⁶²⁶) of the amino acid sequence of COMP (SEQ ID NO: 1).

8. An antibody according to claim 7 for use in diagnosis, differential diagnosis, disease monitoring and/or therapeutic monitoring of a tissue degradation process.

9. A method according to claim 1, wherein the presence of one or more neoepitopes of COMP is detected by using one or more antibodies as defined in claim 7 or 8.

## Patentansprüche

1. Verfahren zum Bestimmen eines Gewebeverschlechterungsprozesses, das das Detektieren des Vorhandenseins in einer Probe eines oder mehrerer Neoepitope eines Säugetier COMPs, die nach der Spaltung von COMP zwischen Position 625 (N⁶²⁵) und 626 (^{P626}) der Aminosäurensequenz von COMP (SEQ ID NR: 1) auftreten, umfasst.

2. Isoliertes C-endig abgeschnittenes Fragment von menschlichem COMP, wobei das C-Ende des Fragments eine Aminosäure 625 von SEQ ID NR: 1 ist und wobei das Fragment eine Aminosäuresequenz enthält, die in irgendeiner der SEQ ID NRn: 22-31 gegeben ist.

3. Isoliertes N-endig abgeschnittenes Fragment von menschlichem COMP, wobei das N-Ende des Fragments eine Aminosäure 626 von SEQ ID NR: 1 ist und wobei das Fragment eine Aminosäuresequenz enthält, die in irgendeiner der SEQ ID NRn: 32-40 gegeben ist.

4. Isoliertes Fragment von menschlichem COMP, das aus der Aminosäuresequenz besteht, die in irgendeiner der SEQ ID NRn: 22-40 gegeben ist.

5. Verwendung eines Fragments von COMP nach einem der Ansprüche 2 bis 4 für die Produktion von Antikörpern gegen ein oder mehrere COMP-Neoepitope, die nach der Spaltung von COMP zwischen Position 625 (N⁶²⁵) und 626 (P⁶²⁶) der Aminosäuresequenz von COMP (SEQ ID NR: 1) auftreten.

6. Konjugation bzw. Konjugat für die Verwendung bei der Produktion von Antikörpern gegen ein oder mehrere COMP-Neoepitope, die nach der Spaltung von COMP zwischen Position 625(N⁶²⁵) und 626(P⁶²⁶) der Aminosäuresequenz von COMP (SEQ ID NR: 1) auftreten, die bzw. das besteht aus (i) einem oder mehreren Fragmenten nach Anspruch 4, die mit einem Proteinträger gekoppelt oder vermischt sind, oder aus (ii) einem oder mehreren Fragmenten nach Anspruch 2 oder Anspruch 3, die über das Ende, das nicht den Spaltungsort repräsentiert, mit einem Proteinträger gekoppelt sind.

7. Antikörper gegen ein isoliertes Fragment von COMP nach einem der Ansprüche 2 bis 4 oder Konjugation nach Anspruch 6, wobei der Antikörper an ein oder mehrere COMP-Neoepitope bindet, die nach der Spaltung von COMP zwischen Position 625(N⁶²⁵) und 626(P⁶²⁶) der Aminosäuresequenz von COMP (SEQ ID NR: 1) auftreten.

8. Antikörper nach Anspruch 7 zur Verwendung bei der Diagnose, der differentiellen Diagnose, der Krankheitsüberwachung und/oder der therapeutischen Überwachung eines Gewebeverschlechterungsprozesses.

9. Verfahren nach Anspruch 1, wobei das Vorhandensein eines oder mehrerer Neoepitope von COMP unter Verwendung eines oder mehrerer Antikörper nach Anspruch 7 oder 8 detektiert wird.

## Revendications

1. Procédé pour déterminer un processus de dégradation tissulaire, comportant l'étape consistant à détecter, dans un échantillon, la présence d'un ou plusieurs néo-épitopes de protéine COMP de mammifère qui apparaissent après clivage de la protéine COMP entre les positions 625(N⁶²⁵) et 626 (P⁶²⁶) de la séquence d'acides aminés de la protéine COMP (SEQ ID N° : 1).

2. Fragment C-terminal tronqué isolé de la protéine COMP humaine, dans lequel l'acide aminé C-terminal du fragment est l'acide aminé 625 de la SEQ ID N° : 1, et dans lequel le fragment comporte une séquence d'acides aminés donnée dans l'une quelconque des SEQ ID N° : 22 à 31.

3. Fragment N-terminal tronqué isolé de la protéine COMP humaine, dans lequel l'acide aminé N-terminal du fragment est l'acide aminé 626 de la SEQ ID N° : 1, et dans lequel le fragment comporte une séquence d'acides aminés donnée dans l'une quelconque des SEQ ID N° : 32 à 40.

4. Fragment isolé de la protéine COMP humaine, constitué de la séquence d'acides aminés donnée dans l'une quelconque des SEQ ID N° : 22 à 40.

5. Utilisation d'un fragment de protéine COMP tel que défini dans l'une quelconque des revendications 2 à 4 pour la production d'anticorps dirigés contre un ou plusieurs néo-épitopes de protéine COMP qui apparaissent après clivage entre les positions 625 (N⁶²⁵) et 626 (P⁶²⁶) de la séquence d'acides aminés de la protéine COMP (SEQ ID N° : 1).

6. Conjugué pour une utilisation dans la production d'anticorps dirigés contre un ou plusieurs néo-épitopes de protéine COMP qui apparaissent après clivage de la protéine COMP entre les positions 625 (N⁶²⁵) et 626 (P⁶²⁶) de la séquence d'acides aminés de la protéine COMP (SEQ ID N° : 1), constitué de (i) un ou plusieurs fragments tels que définis dans la revendication 4 couplés ou mélangés à une protéine porteuse, ou (ii) un ou plusieurs fragments tels que définis dans la revendication 2 ou la revendication 3, couplés via leur extrémité qui ne représente pas le site de clivage à une protéine porteuse.

7. Anticorps dirigé contre un fragment isolé de protéine COMP tel que revendiqué dans l'une quelconque des revendications 2 à 4 ou un conjugué tel que revendiqué dans la revendication 6, dans lequel l'anticorps se lie à un ou plusieurs néo-épitopes de protéine COMP qui apparaissent après clivage de la protéine COMP entre les positions 625 (N⁶²⁵) et 626 (P⁶²⁶) de la séquence d'acides aminés de la protéine COMP (SEQ ID N° : 1).

8. Anticorps selon la revendication 7 pour une utilisation dans le diagnostic, le diagnostic différentiel, la surveillance de maladie et/ou la surveillance thérapeutique d'un processus de dégradation tissulaire.

9. Procédé selon la revendication 1, dans lequel la présence d'un ou plusieurs néo-épitopes de la protéine COMP est détectée en utilisant un ou plusieurs anticorps tels que définis dans la revendication 7 ou 8.


### SEQUENCE LISTING

<110> AnaMar Medical AB
<120> Method for determining a tissue degradation precess by detection of COMP neoepitopes
<130> P11314 PC/P10859
<160> 41
<170> PatentIn version 3.1
<210> 1
   <211> 737
   <212> PRT
   <213> Homo sapiens
<400> 1
<210> 2
   <211> 12
   <212> PRT
   <213> Homo sapiens
<400> 2
   Leu Asp Pro Glu Gly Asp Ala Gln Ile Asp Pro Asn
   1 5 10
<210> 3
   <211> 11
   <212> PRT
   <213> Homo sapiens
<400> 3
   Asp Pro Glu Gly Asp Ala Gln Ile Asp Pro Asn
   1 5 10
<210> 4
   <211> 10
   <212> PRT
   <213> Homo sapiens
<400> 4
   Pro Glu Gly Asp Ala Gln Ile Asp Pro Asn
   1 5 10
<210> 5
   <211> 9
   <212> PRT
   <213> Homo sapiens
<400> 5
   Glu Gly Asp Ala Gln Ile Asp Pro Asn
   1               5
<210> 6
   <211> 8
   <212> PRT
   <213> Homo sapiens
<400> 6
   Gly Asp Ala Gln Ile Asp Pro Asn
   1               5
<210> 7
   <211> 7
   <212> PRT
   <213> Homo sapiens
<400> 7
   Asp Ala Gln Ile Asp Pro Asn
   1               5
<210> 8
   <211> 6
   <212> PRT
   <213> Homo sapiens
<400> 8
   Ala Gln Ile Asp Pro Asn
   1               5
<210> 9
   <211> 5
   <212> PRT
   <213> Homo sapiens
<400> 9
   Gln Ile Asp Pro Asn
   1               5
<210> 10
   <211> 4
   <212> PRT
   <213> Homo sapiens
<400> 10
   Ile Asp Pro Asn
   1
<210> 11
   <211> 3
   <212> PRT
   <213> Homo sapiens
<400> 11
   Asp Pro Asn
   1
<210> 12
   <211> 12
   <212> PRT
   <213> Homo sapiens
<400> 12
   Trp Val Val Leu Asn Gln Gly Arg Glu Ile Val Gln
   1 5 10
<210> 13
   <211> 11
   <212> PRT
   <213> Homo sapiens
<400> 13
   Trp Val Val Leu Asn Gln Gly Arg Glu Ile Val
   1 5 10
<210> 14
   <211> 10
   <212> PRT
   <213> Homo sapiens
<400> 14
   Trp Val Val Leu Asn Gln Gly Arg Glu Ile
   1 5 10
<210> 15
   <211> 9
   <212> PRT
   <213> Homo sapiens
<400> 15
   Trp Val Val Leu Asn Gln Gly Arg Glu
   1 5
<210> 16
   <211> 8
   <212> PRT
   <213> Homo sapiens
<400> 16
   Trp Val Val Leu Asn Gln Gly Arg
   1               5
<210> 17
   <211> 7
   <212> PRT
   <213> Homo sapiens
<400> 17
   Trp Val Val Leu Asn Gln Gly
   1               5
<210> 18
   <211> 6
   <212> PRT
   <213> Homo sapiens
<400> 18
   Trp Val Val Leu Asn Gln
   1               5
<210> 19
   <211> 5
   <212> PRT
   <213> Homo sapiens
<400> 19
   Trp Val Val Leu Asn
   1               5
<210> 20
   <211> 4
   <212> PRT
   <213> Homo sapiens
<400> 20
   Trp Val Val Leu
   1
<210> 21
   <211> 3
   <212> PRT
   <213> Homo sapiens
<400> 21
   Trp Val Val
   1
<210> 22
   <211> 12
   <212> PRT
   <213> Homo sapiens
<400> 22
   Trp Lys Gln Met Glu Gln Thr Tyr Trp Gln Ala Asn
   1 5 10
<210> 23
   <211> 11
   <212> PRT
   <213> Homo sapiens
<400> 23
   Lys Gln Met Glu Gln Thr Tyr Trp Gln Ala Asn
   1 5 10
<210> 24
   <211> 10
   <212> PRT
   <213> Homo sapiens
<400> 24
   Gln Met Glu Gln Thr Tyr Trp Gln Ala Asn
   1 5 10
<210> 25
   <211> 9
   <212> PRT
   <213> Homo sapiens
<400> 25
   Met Glu Gln Thr Tyr Trp Gln Ala Asn
   1 5
<210> 26
   <211> 8
   <212> PRT
   <213> Homo sapiens
<400> 26
   Glu Gln Thr Tyr Trp Gln Ala Asn
   1 5
<210> 27
   <211> 7
   <212> PRT
   <213> Homo sapiens
<400> 27
   Gln Thr Tyr Trp Gln Ala Asn
   1 5
<210> 28
   <211> 6
   <212> PRT
   <213> Homo sapiens
<400> 28
   Thr Tyr Trp Gln Ala Asn
   1 5
<210> 29
   <211> 5
   <212> PRT
   <213> Homo sapiens
<400> 29
   Tyr Trp Gln Ala Asn
   1 5
<210> 30
   <211> 4
   <212> PRT
   <213> Homo sapiens
<400> 30
   Trp Gln Ala Asn
   1
<210> 31
   <211> 3
   <212> PRT
   <213> Homo sapiens
<400> 31
   Gln Ala Asn
   1
<210> 32
   <211> 12
   <212> PRT
   <213> Homo sapiens
<400> 32
   Pro Phe Arg Ala Val Ala Glu Pro Gly Ile Gln Leu
   1 5 10
<210> 33
   <211> 11
   <212> PRT
   <213> Homo sapiens
<400> 33
   Pro Phe Arg Ala Val Ala Glu Pro Gly Ile Gln
   1 5 10
<210> 34
   <211> 10
   <212> PRT
   <213> Homo sapiens
<400> 34
   Pro Phe Arg Ala Val Ala Glu Pro Gly Ile
   1 5 10
<210> 35
   <211> 9
   <212> PRT
   <213> Homo sapiens
<400> 35
   Pro Phe Arg Ala Val Ala Glu Pro Gly
   1 5
<210> 36
   <211> 8
   <212> PRT
   <213> Homo sapiens
<400> 36
   Pro Phe Arg Ala Val Ala Glu Pro
   1 5
<210> 37
   <211> 7
   <212> PRT
   <213> Homo sapiens
<400> 37
   Pro Phe Arg Ala Val Ala Glu
   1               5
<210> 38
   <211> 6
   <212> PRT
   <213> Homo sapiens
<400> 38
   Pro Phe Arg Ala Val Ala
   1               5
<210> 39
   <211> 5
   <212> PRT
   <213> Homo sapiens
<400> 39
   Pro Phe Arg Ala Val
   1               5
<210> 40
   <211> 4
   <212> PRT
   <213> Homo sapiens
<400> 40
   Pro Phe Arg Ala
   1
<210> 41
   <211> 3
   <212> PRT
   <213> Homo sapiens
<400> 41
   Pro Phe Arg
   1